(19) Europäisches Patentamt
European Patent Office
Office européen des brevets

(11) Numéro de publication: **0 192 565 B1**

# (12) FASCICULE DE BREVET EUROPEEN

(45) Date de publication de fascicule du brevet: **02.05.91**

(21) Numéro de dépôt: **86400305.8**

(22) Date de dépôt: **12.02.86**

Le dossier contient des informations techniques présentées postérieurement au dépôt de la demande et ne figurant pas dans le présent fascicule.

(51) Int. Cl.⁵: **G01N 33/53**, G01N 33/577, G01N 33/543, G01N 33/74, C07K 15/00, C12P 21/00, C12N 5/00, //(C12P21/00, C12R1:91)

## (54) Procédé de dosage immunologique des amines, anticorps monoclonaux et ensemble de réactifs pour la mise en oeuvre du procédé.

(30) Priorité: **14.02.85 FR 8502130**

(43) Date de publication de la demande: **27.08.86 Bulletin 86/35**

(45) Mention de la délivrance du brevet: **02.05.91 Bulletin 91/18**

(84) Etats contractants désignés: **AT BE CH DE FR GB IT LI LU NL SE**

(56) Documents cités:
**EP-A- 0 110 640**
**FR-A- 2 334 107**
**FR-A- 2 523 311**

**CHEMICAL ABSTRACTS, vol. 101, no. 3, 16 juillet 1984, page 564, no. 23070r, Columbus, Ohio, US; C.R. TINDALE et al.: "Reactions of biogenic amines with quinones"**

(73) Titulaire: **INSTITUT PASTEUR**
**25-28, rue du Docteur Roux**
**F-75724 Paris Cédex 15(FR)**

Titulaire: **CENTRE NATIONAL DE LA RECHERCHE SCIENTIFIQUE (CNRS)**
**15, Quai Anatole France**
**F-75007 Paris(FR)**

(72) Inventeur: **Guesdon, Jean-Luc**
**12 rue du Hameau**
**F-75015 Paris(FR)**
Inventeur: **Avrameas, Stratis**
**1 rue Sarasate**
**F-75015 Paris(FR)**
Inventeur: **Mazie, Jean-Claude**
**18 rue Gramme**
**F-75011 Paris(FR)**

EP 0 192 565 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**CHEMICAL ABSTRACTS, vol. 78, no. 7, 19 février 1973, page 175, no. 39677m, Columbus, Ohio, US; G.H. MOXON et al.: "Complexing of histamine with p-benzoquinone"**

**CHEMICAL ABSTRACTS, vol. 101, no. 1, 2 juillet 1984, page 457, no. 5266e, Columbus, Ohio, US; H. MITA et al.: "An attempt to produce an antibody to histamine and histamine derivatives"**

(74) Mandataire: **Phélip, Bruno et al**
**c/o Cabinet Harlé & Phélip 21, rue de La Rochefoucauld**
**F-75009 Paris(FR)**

## Description

La présente invention concerne un procédé de dosage immunologique des amines présentes dans les milieux biologiques, les anticorps monoclonaux spécifiques des produits résultant de la réaction de l'amine sur une quinone. les hybridomes secrétant ces anticorps et les coffrets contenant les réactifs nécessaires à la mise en oeuvre du procédé de dosage.

Le dosage de l'histamine est l'un des tests les plus significatifs de l'hypersensibilité immédiate. Plusieurs méthodes de dosage de l'histamine sont actuellement disponibles mais ne peuvent être réalisées que dans des laboratoires possédant un appareillage coûteux.

La méthode la plus couramment utilisée en clinique est basée sur un dosage fluorimétrique comportant une extraction par le butanol du sérum à étudier, suivie d'un traitement par une solution aqueuse d'acide chlorhydrique contenant de l'heptane ; l'histamine prélevée dans la phase chlorhydrique est condensée avec de l'aldéhyde o-phtalique, en milieu alcalin, pour former un composé fluorescent ; la fluorescence mesurée est proportionnelle à la quantité d'histamine présente dans le milieu, et qui avait été libérée par les basophiles ou les mastocytes. Cette technique est longue et délicate même lorsqu'elle est automatisée.

La méthode radio-enzymatique aussi pratiquée, pose des problèmes de spécificité ; ainsi par exemple, la chlorpromazine et la sérotonine inhibent l'activité enzymatique de l'histamine-N-méthyl-transférase.

Une autre méthode utilise la chromatographie en phase gazeuse couplée à la spectrométrie de masse et implique donc l'utilisation d'appareils complexes et particulièrement coûteux.

Il serait ainsi particulièrement avantageux de pouvoir doser l'histamine par une méthode immunologique, puisqu'on éviterait ces inconvénients. En effet une telle méthode étant spécifique, il ne serait pas nécessaire de procéder à une extraction chimique de l'histamine et par ailleurs la méthode serait sensible puisque l'on sait marquer les anticorps par des substances faciles à doser même en très faible quantité, comme avec des enzymes, des radio-isotopes, des fluorochromes.

Il est malheureusement apparu particulièrement difficile d'obtenir des anticorps spécifiques de l'histamine et récemment H. Mita et coll. ont indiqué qu'ils n'avaient pas pu obtenir des anticorps dirigés contre l'histamine, ou contre des conjugués histamine/sérum albumine bovine chez le lapin, dans : Agents and Actions 14 p. 574-579 (1984).

On a maintenant trouvé qu'en conjuguant l'histamine à un composé de faible masse moléculaire, une quinone, on pouvait obtenir des anticorps spécifiques dudit composé d'addition, utilisables dans un procédé de dosage immunologique de l'histamine.

Dans ce qui suit, on se réfèrera à l'histamine, mais il apparaîtra au spécialiste que le procédé d'obtention des anticorps et la mise en oeuvre de ceux-ci, est aussi applicable à d'autres amines présentes dans les milieux biologiques et susceptibles de s'additionner sur une quinone, c'est-à-dire des amines primaires ou secondaires.

Parmi celles-ci, on peut citer les neurotransmetteurs, tels que la dopamine ou la norépinéphrine, les acides aminés, tels que la glycine ou le glutamate, les peptides de faible masse, tels que l'encéphaline, la substance P ou la neurotensine.

Le procédé de dosage immunologique des amines dans les milieux biologiques, qui fait l'objet de la présente invention, est caractérisé par le fait que :

a) on fait réagir, avec une quinone, l'amine présente dans l'échantillon à étudier ;

b) on introduit ensuite dans le milieu un anticorps monoclonal reconnaissant spécifiquement le produit de réaction amine-quinone formé ; et

c) on dose l'une des formes de l'anticorps monoclonal présent.

Parmi les quinones pouvant convenir, figurent les naphtoquinone, pyrènequinone, pérylènequinone, diphénoquinone, et les benzoquinones. On préfère tout particulièrement la p-benzoquinone qui comme les cétones $\alpha,\beta$-éthyléniques peut former des produits d'addition 1,4.

Par anticorps monoclonal spécifique du produit de réaction amine-quinone, on entend un anticorps qui complexe ce produit, sous sa forme libre ou immobilisée, sans complexer l'amine seule, ni la quinone, que celle-ci soit libre ou immobilisée comme on le décrira ci-après, cet anticorps ne se fixant pas non plus, dans le cas d'une quinone immobilisée, sur la substance utilisée pour le couplage de ladite quinone à son support insoluble, ni sur ledit support, comme cela sera décrit ci-après.

Il est particulièrement surprenant de pouvoir obtenir de tels anticorps monoclonaux qui reconnais sent le produit résultant de la réaction de la quinone sur une amine, sans reconnaître chacune de ces structures séparées.

Ces anticorps monoclonaux sont sécrétés par des hybridomes, préparés selon la technique connue décrite pour la première fois par Köhler et Milstein dans Nature, 256 , 495-97 (1975). Pour obtenir les cellules qui seront fusionnées avec des cellules de myélome murin, on injecte à des souris un conjugué

protéine/amine à étudier, préparé en faisant réagir la quinone considérée sur une protéine, puis l'amine primaire sur la protéine "sensibilisée" ainsi obtenue. Le couplage de différents composés de forte masse moléculaire aux protéines par l'intermédiaire d'une quinone est décrit dans différents articles de S.AVRAMEAS et T. TERNYNCK, dont notamment Immunochemistry vol. 14 p. 767 (1977) ; la même méthode est appliquée dans ce cas.

La protéine utilisée pour préparer le conjugué immunogène peut être par exemple l'albumine bovine, l'ovalbumine, une enzyme telle que la glucose-oxydase, la ribonucléase, ou encore l'hémocyanine de lamproie.

Les hybridomes obtenus par fusion des cellules de myélome murin, telles que SP-2/0 ou Ag 8 x 63 - 653, et les cellules de rate des souris immunisées, sont sélectionnés par clonage et sous-clonage de telle sorte qu'on isole les lignées d'hybridomes secréteurs d'anticorps monoclonaux ayant une forte affinité pour le composé d'addition 1,4 du type de ceux de formules I ou II ci-après représentées, et une affinité négligeable pour l'amine libre, la quinone libre ou immobilisée, ainsi que pour la polyamine utilisée pour le couplage de la quinone au support insoluble.

L'immunoréactivité des surnageants de culture est contrôlée par une technique enzymo-immunologique directe. Tout d'abord, chaque surnageant est testé simultanément vis-à-vis du conjugué histamine-quinone-polyamine et du conjugué histidine-quinone-polyamine. La présence d'anticorps liés à ces deux conjugués est révélée par des anticorps anti-IgG de souris marqués par une enzyme. Les hybridomes qui secrètent des anticorps qui se combinent uniquement avec le premier conjugué sont seuls conservés. La spécificité fine de ces anticorps est ensuite analysée par une technique d'inhibition en utilisant comme inhibiteur la quinone, le composé histamine-quinone, le composé histidine-quinone, le composé glycine-quinone, la polyamine, l'histidine, l'histamine, les conjugués histamine-quinone-polyamine, histidine-quinone-polyamine et glycine-quinone-polyamine. Les hybridomes sélectionnés sont ceux qui secrètent des anticorps reconnaissant uniquement le composé histamine-quinone. Par composé histamine-quinone (ou histidine, ou glycine), on entend le composé résultant de la réaction de l'histamine sur la quinone (ou de l'histidine, ou de la glycine). Par conjugué histamine-quinone-polyamine, on entend le résultat de la réaction de la quinone avec le polyamine suivie de la réaction du produit obtenu avec de l'histamine.

Dans le cas de la recherche d'anticorps monoclonaux dirigés contre une autre amine que l'histamine, on pourra utiliser pour la sélection d'autres amines que l'histidine ou la glycine, c'est-à-dire d'autres analogues structuraux de l'amine considérée.

Les hybridomes secrétant des anticorps monoclonaux ayant les caractéristiques d'affinité précédemment définies ainsi que ces anticorps monoclonaux sont un autre objet de l'invention.

Des échantillons de l'hybridome préféré préparé à ce jour, ont été déposés à la Collection Nationale de Microorganismes à l'Institut Pasteur, Paris, le 4 février 1985, sous le n° I-421.

Conformément à la présente invention, entre l'étape a) et l'étape b) du procédé sus-indiqué, on effectue notamment le blocage des fonctions réactives restantes de la quinone. On effectue ce blocage en faisant réagir le composé résultant de la réaction de la quinone et de l'amine, avec une amine primaire en excès, telle que la glycine, la lysine, l'éthanolamine, le trishydroxyméthylaminométhane, dit TRIS, ou analogues.

Conformément à un premier mode de réalisation du procédé selon la présente invention, à l'étape a), on met en oeuvre la quinone à l'état immobilisé sur un support insoluble par l'intermédiaire d'un polymère à fonction amine et à l'étape c), on dose l'anticorps monoclonal présent dans sa forme complexée au produit de réaction amine-quinone.

Par quinone immobilisée, on entend la quinone qui a réagi sur une polyamine - telle qu'une protéine, un polypeptide, un polyéthylèneimine - préalablement fixée sur un support insoluble - tel qu'une plaque de microtitration, les parois de tubes ou billes en polystyrène, polypropylène, chlorure de polyvinyle, polyacrylamide et analogues, les gels de polyacrylamide, d'agarose ou de polyacrylamide/ agarose. Dans le cas de la p-benzoquinone, le schéma réactionnel est le suivant :

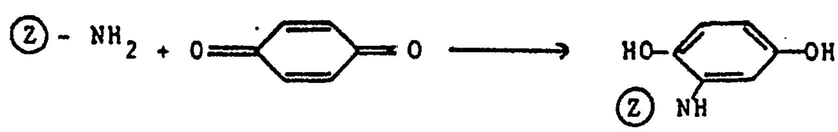

dans lequel Z représente la polyamine primaire - dont une seule fonction amine est mise en évidence - fixée sur un support. Lorsque l'amine à doser réagit sur la quinone, on obtient un composé d'addition 1,4 ; dans le cas de la p-benzoquinone libre et d'une amine primaire, représentée par $RNH_2$, le schéma réactionnel est le suivant :

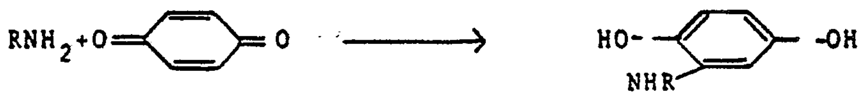

I

tandis que lorsque $RNH_2$ réagit sur la p-benzoquinone immobilisée, le produit d'addition est représenté par la formule suivante :

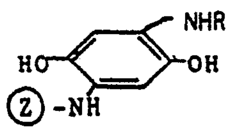

II

Dans le cas du premier mode de réalisation précité du procédé selon l'invention, à l'étape c) une technique particulièrement sensible consiste à faire réagir sur l'anticorps monoclonal présent dans sa forme complexée, un anticorps dirigé contre lui et marqué par un élément dosable, et, après réaction, on détermine la quantité dudit anticorps marqué qui s'est fixé audit anticorps monoclonal.

Parmi les agents de marquage connus des anticorps, on peut citer les éléments radioactifs, les éléments fluorescents, les enzymes, les globules rouges. On préfère les enzymes, telles que la galactosidase, la peroxydase, la glucose-oxydase, la phosphatase alcaline. Cette technique peut être qualifiée de dosage immunologique direct.

Conformément à un seconde mode de réalisation du procédé selon la présente invention, à l'étape a), on met en oeuvre la quinone à l'état libre et à l'étape c), on dose l'anticorps monoclonal dans sa forme libre.

Dans ce cas , on peut notamment procéder de deux façons différentes pour exécuter l'étape c):

- dans une première variante,à cette étage c),on introduit, dans le milieu,une quantité connue du composé résultant de la réaction de l'amine considérée et de la quinone considérée,immobilisée sur un support insoluble par l'intermédiaire d'un polymère à fonctions amine,ainsi qu'un anticorps dirigé contre l'anticorps monoclonal et marqué par un élément dosable,et, après réaction on détermine la quantité de l'anticorps marqué qui s'est fixé sur ledit support portant le produit de réaction quinone-amine.
- dans une seconde variante,à cette étape c), l'anticorps monoclonal est de préférence immobilisé sur un support et on ajoute au milieu un conjugé enzyme-amine et on dose l'activité enzymatique soit sur le support,soit en phase liquide.On utilise , de préférence,comme enzyme,une peroxydase.

Les échantillons dans lesquels on dosera par le procédé de l'invention les amines,peuvent être préparés à partir des tissus et liquides biologiques,de façon connue en soi et dilués si nécessaire de telle sorte que la concentration des amines soit en général comprise entre 10 pg/ml et 20 ng/ml;cette concentration est fonction du pH du milieu réactionnel,de l'affinité des agents immunogéniques en présence, de la quantité de quinone fixée au support et de la nature du marquage de l'anticorps utilisé pour la révélation.

Toutefois, la mise en oeuvre de l'étape c) conformément à la seconde variante,utilisant un conjugué "amine-enzyme",le couplage amine/enzyme impliquant la conservation de l'activité enzymatique,permet de doser notamment l'histamine de façon simple et reproductible lorsque celle-ci se trouve dans un milieu biologique complexe (par exemple dans le sang total).

Dans ce qui suit on décrit des exemples de mise en oeuvre de l'invention:

EXEMPLE 1:Obtention d'hybridomes secréteurs d'anticorps monoclonaux spécifiques du composé d'addition 1,4 formé par action de la para-benzoquinone sur l'histamine .

1) Préparation de l'immunogène :

On dissout 10 mg d'albumine bovine dans 2 ml de tampon phosphate (0,1 M ; pH = 6,0) et on ajoute 30 mg de p-benzoquinone dissous dans 0,6 ml d'éthanol. Après une heure sous agitation à température ambiante, la solution est chromatogrophiée sur une colonne de Trisacryl GF05, commercialisée par IBF - Pharmindustrie, qui est à base de copolymères acryliques ; la colonne contenant environ 50 ml de gel avait été préalablement équilibrée avec une solution aqueuse de chlorure de sodium 0,15 M. On recueille en

éluant avec la même solution de chlorure de sodium la fraction de tête jusqu'a un volume correspondant au volume mort de la colonne. On ajoute à cette fraction la quantité suffisante de solution aqueuse de bicarbonate de sodium 1M pour obtenir une concentration finale en bicarbonate de 0,1 M, puis on introduit 10 mg d'histamine. Après 24 heures à 18° C, la solution est chromatographiée sur une colonne constituée d'environ 50 ml de Trisacryl GF05, équilibrée avec une solution de chlorure de sodium 0,15 M, tamponnée à pH 7,4 avec du phosphate de potassium 0,01 M ; l'élution est réalisée avec cette même solution. L'immunogène ainsi obtenu contient en moyenne 2,6 molécules d'histamine par molécule d'albumine.

2) Immunisation des souris

On pratique 4 injections, y compris l'injection de rappel, de l'immunogène aux animaux à raison de 100 µg d'antigène dans 200 µl d'adjuvant complet de Freund par voie sous-cutanée, pour les deux premières injections à 14 jours d'intervalle et de 50µg d'antigène dans 200 µl de sérum physiologique par voie intraveineuse pour les deux suivantes pratiquées le même jour et 4 jours après la seconde.

Le sacrifice et l'isolement des cellules spléniques a lieu 3 jours après la dernière injection.

3) Fusion

La fusion avec des cellules d'hybridome adaptées SP 2/0, est réalisée en présence de polyéthylène glycol 1000, commercialisé par la Société MERCK.

4) Sélection des hybridomes

La culture des clones et sous-clones est effectuée sur le milieu RPMI 1640, complémenté par du sérum de cheval à 10 %; la composition de ce milieu classique est mentionnée dans In Vitro 9, p. 6 (1974). On sélectionne les clones en étudiant le contenu des surnageants des puits de culture par la méthode dite ELISA (enzyme linked immunosorbent assay), c'est-à-dire "essai utilisant une enzyme fixée à un immunoadsorbant".

5) Sélection

A partir de cultures primaires, en utilisant la technique d'analyse des surnageants précédemment décrite, on a ainsi isolé 2 clones dont l'affinité pour le conjugué histamine/p-benzoquinone/albumine est nettement supérieure à celle pour l'histamine libre.

Pour le clone de référenceD22-12, déposé à la CNCM sous le n° I-421, les affinités relatives de l'anticorps monoclonal secrété figurent dans le tableau 1.

TABLEAU 1

| Nature de l'antigène | affinité relative |
|---|---|
| Conjugué histamine/ p-benzoquinone/albumine | 1 |
| histamine libre | $0,5 \times 10^{-5}$ |
| conjugué p-benzoquinone/albumine | - ∞ |
| albumine seule | - ∞ |

EXEMPLE 2 : Dosage de l'histamine de type "direct" .

1) Préparation de la p-benzoquinone immobilisée .

On introduit 100 µl d'une solution d'albumine bovine à 10 µg/ml dans un tampon phosphate salin (PBS) contenant 0,02 % (p/V) d'azoture de sodium, dans chaque puits d'une microplaque de titration en polystyrène. La plaque est couverte puis incubée pendant 2 heures à 60° C et au moins une nuit à 4° C. Le liquide est éliminé des puits qui sont ensuite lavés par une solution de phosphate de sodium (0,1M; pH = 4,5).

On introduit ensuite dans chacun des puits ainsi traités 100 µl d'une solution de para-benzoquinone à 3 mg/ml dans un mélange à 10 % (V/V) d'éthanol et de phosphate de potassium (0,1 M ; pH = 4,5). La plaque couverte, est maintenue 1 heure à 4° C, à l'obscurité. Le liquide est décanté et les puits sont lavés avec du tampon phosphate (0,1 M ; pH = 4,5) pour éliminer l'excès de benzoquinone.

2) Fixation de l'histamine à doser

L'échantillon (témoin ou à étudier) est dilué avec un tampon bicarbonate de sodium (0,1 M) pour obtenir un pH = 8,5, de telle sorte que l'on ait une concentration en histamine comprise entre 0,1 et 20 ng/ml.

On introduit alors 100 µl de cette dilution dans les puits de la plaque précédemment obtenus et l'on maintient 4 heures à 37° C, la plaque couverte. On élimine alors le liquide des puits et les lave par une solution dénommée TBS constituée d'une solution aqueuse, de TRIS (0,01 M ; pH = 7,4 ) et de NaCl (0,15 M) dans laquelle on a introduit un agent tensio-actif classique,le TWEEN 20, à raison de 0:1%.

3) Complexation.

Pour obtenir de grandes quantités d'anticorps monoclonal, on a appliqué une méthode connue : formation d'ascites chez des souris auxquelles on injecte par voie intrapéritonéale les anticorps monoclonaux secrétés par l'hybridome préféré isolé dans l'exemple 1 et isolement du liquide d'ascite. Les anticorps sont dilués dans du TBS contenant 1 % d'albumine bovine et 0,1 % de TWEEN 20 pour obtenir des solutions de concentration voisine de 1 µg/ml

On introduit dans chacun des puits précédemment traités 100 µl de dilution d'anticorps monoclonal et laisse la plaque 1 nuit environ à la température ambiante. On élimine alors le liquide des puits et les lave avec une solution de TBS contenant 0,1 % de TWEEN 20.

4) Révélation

a) Des anticorps anti-IgG de souris, marqués à la β-galactosidase sont dilués dans une solution de TBS contenant 0,02 % (p/V) d'azoture de sodium pour obtenir des concentrations en anticorps comprises entre 1 à 5 µg/ml. On en introduit 100 µl dans chaque puits et abandonne la plaque couverte, 2 heures à température ambiante avant d'éliminer le liquide des puits et de les laver avec du TBS contenant 0,1 % de TWEEN 20.

b) On introduit ensuite dans chacun des puits 200 µl d'une solution aqueuse tamponnée d'un substrat spécifique de la β-galactosidase, l'ortho-nitrophényl galactopyranoside, de concentration 0,8 mg/ml. Après 2 à 4 heures à 37° C, on arrête la réaction enzymatique par addition de 50 µl de solution aqueuse de $Na_2CO_3$ (2M) et on détermine par lecture de la densité optique de la solution contenue dans les micropuits, la quantité de substrat transformée. Sur la figure 1 est représentée, en coordonnées semi-logarithmiques,la densité optique lue, à 414 nm, en fonction de la concentration après dilution, en histamine de l'échantillon de départ, exprimée en ng d'histamine par ml.

On constate que dans ces conditions le seuil de détection de l'histamine est voisin de 20 pg/100 µ1.

EXEMPLE 3 : Dosage de l'histamine par la méthode enzymoimmunométrique.

1) Réaction de l'amine sur la p-benzoquinone.

On mélange dans des petits tubes (volume à volume) l'échantillon à doser ou l'échantillon témoin, contenant l'histamine avec une solution de p-benzoquinone à 6 mg/ml dans un tampon phosphate (0,1 M ; pH = 4,5) contenant 20 % (V/V) d'éthanol pour obtenir une solution ayant un volume final de 0,5 ml environ. Le mélange est abandonné à température ambiante 1 heure à l'obscurité.

2) Blocage des fonctions réactives.

On introduit ensuite dans chacun des tubes 50 µl d'une solution aqueuse de glycine (2M) et carbonate de sodium (2M), pour obtenir un pH final compris entre 7,5 et 8,5 et abandonne le mélange à température ambiante 1 heure à l'obscurité.

3) Complexation.

On introduit alors 90 µl d'une solution d'anticorps monoclonal, précédemment obtenu, dans une solution aqueuse de phosphate monopotassique (0,5 M) contenant 10 % (p/V) de sérum albumine bovine et 0,1 % de TWEEN 20, de telle sorte que la concentration en anticorps soit voisine de 100 ng/ml.

Les tubes sont abandonnés à température ambiante pour 20 heures.

4) Révélation

Les solutions contenues dans les tubes sont alors introduites dans les micropuits d'une plaque sur les parois desquels ont été fixés de l'histamine par l'intermédiaire de la sérum albumine bovine comme décrit à l'exemple 2-1 et 2-2,à raison de 1 µg d'histamine par puits.

Dans chacun des puits ainsi remplis, on ajoute ensuite une solution d'anticorps marqués anti-IgG de souris dans les conditions décrites à l'exemple 1 (4-a) et on révèle comme à l'exemple 1 (4-b).

L'activité enzymatique mesurée est pratiquement inversement proportionnelle à la concentration de l'échantillon de départ en histamine, comme on peut le constater sur la figure 2 sur laquelle est portée la densité optique lue en fonction de la concentration d'histamine dans la solution.

Le seuil de détection avec les anticorps monoclonaux secrétés par les hybridomes de référence D22-12 dans ces conditions est donc de 5 ng/ml.

EXEMPLE 4 : Comparaison des résultats obtenus avec la méthode directe selon l'invention et une méthode fluorimétrique connue.

On a dosé successivement par les deux méthodes, 22 échantillons de solutions contenant de l'histamine à diverses concentrations. Ces solutions ont été obtenues à partir de lavages de poumons de cobaye sensibilisés avec l'ovalbumine. Sur la figure 3, on a porté, en abscisses, les résultats obtenus avec la méthode selon l'invention de type direct (E) et en ordonnées avec la méthode fluorimétrique (F) ; on constate sur cette figure qu'il y a corrélation entre les résultats obtenus par les deux méthodes. Sur cette figure 3. les "X" représentent une série d'expériences, et les "⊗" une autre série d'expériences réalisées une semaine plus tard avec des échantillons différents.

EXEMPLE 5 : Préparation du réactif peroxydase-histamine

a) 10 mg de peroxydase de raifort sont dissous dans 1,7 ml de tampon phosphate 0,1M, pH 7,0. Ensuite, on ajoute 0.3 ml d'une solution contenant 30 mg de benzoquinone-1,4 dans 1 ml d'éthanol. On laisse réagir à l'obscurité, pendant 1 heure, à température ambiante.

La peroxydase, liée de manière covalente à la benzoquinone-1,4, est séparée de la benzoquinone qui n'a pas réagi par filtration sur gel (colonne de 2,5 x 8 cm Trisacryl GF 05), suivie d'un lavage avec une solution de NaCl 0,15 M. La première fraction brune est collectée et mélangée à 0,5 ml de tampon borate de sodium-phosphate de potassium, pH 9,0, selon Kolthoff (1932), contenant. 100 mg de

dihydrochlorure d'histamine. Le pH est ajusté à 8,5 par addition d'un volume de tampon borate de sodium saturé. Le mélange est laissé à température ambiante, pendant 20 heures à l'obscurité, puis dialysé contre du tampon TBS froid pendant 24 heures.

b) Une variante du mode opératoire a) ci-dessus consiste à ajouter 0,01 ml de solution alcoolique de benzoquinone-1,4, (10 mg/ml) dans une solution contenant 0,1 mg de dihydrochlorure d'histamine (dissoute dans 0,1 ml de tampon 0,1M de phosphate de potassium à pH 4,5).

Le mélange est laissé pendant 1 heure à température ambiante à l'obscurité. On ajoute ensuite 0,5 ml de solution de peroxydase de raifort (10 mg dissous dans 0,5 ml de tampon borate de sodium-phosphate de potassium, pH 9). Le mélange est laissé pendant une nuit à température ambiante, puis est passé sur une colonne de chromatographie sur Trisacryl GF 05 et la première fraction marron est collectée.

EXEMPLE 6 : Dosade de l'histamine selon l'invention, par la méthode de compétition

a) Réaction de l'histamine avec la benzoquinone

Le couplage histamine-benzoquinone-1,4 a été décrit à l'Exemple 3 ci-dessus.

Après blocage des fonctions réactives et neutralisation selon l'Exemple 3, on procède à la réaction de complexation.

b) Complexation et compétition

L'histamine est fixée sur les anticorps monoclonaux immobilisés selon le mode opératoire suivant :

On met 0.1 ml d'une solution d'histamine traitée à la benzoquinone dans chaque puits de la microplaque sur les parois duquel ont été fixés les anticorps monoclonaux selon l'invention. La plaque est incubée pendant une nuit, à la température ambiante, puis on ajoute 0,1 ml d'une solution de conjugué histamine-peroxydase, tampon "TBS-TWEEN 20 (0,1% volume/volume) )-BSA (1%)."

Après une incubation de 2 heures à la température ambiante, la plaque est lavée trois fois avec du tampon TBS-TWEEN 20 (à 0, 1% en volume de TWEEN 20).

Enfin, l'activité peroxydasique associée à la phase solide de la microplaque est mesurée par addition de 0,2 ml d'un tampon citrate 0,05 M contenant 1 mg/ml d'orthophénylènediamine et 0,06% d'eau oxygénée.

Après 10 minutes, la réaction enzymatique est arrêtée par addition de 0,05 ml d'acide sulfurique 2N contenant 0,5% de $Na_2SO_3$. La lecture est faite à 492 nm.

Sur la figure 4, est représentée, en coordonnées semi-logarithmiques, la densité optique lue, à 492 nm, en fonction de la concentration, après dilution, en histamine de l'échantillon de départ, exprimée en ng d'histamine par ml.

c) Comparaison entre les résultats obtenus par la méthode de compétition et ceux obtenus par une méthode fluorimétrique connue (dosage de l'histamine à partir du sang humain total)

Sur l'axe des ordonnées (y) de la figure 5 est portée la mesure faite selon la méthode par compétition (ELISA) et sur l'axe des abscisses (x) est portée la mesure obtenue par fluorescence. On constate sur cette figure qu'il y a corrélation entre les résultats obtenus par les deux méthodes.

## Revendications

1. Procédé de dosage immunologique des amines dans les milieux biologiques, caractérisé par le fait que :

   a) on fait réagir, avec une quinone, l'amine présente dans l'échantillon à étudier ;

   b) on introduit ensuite dans le milieu un anticorps monoclonal reconnaissant spécifiquement le produit de réaction amine-quinone formé ; et

   c) on dose l'une des formes de l'anticorps monoclonal présent.

2. Procédé selon la revendication 1, caractérisé par le fait qu'entre l'étape a) et l'étape b), on effectue le blocage des fonctions réactives restantes de la quinone.

3. Procédé selon l'une des revendications 1 et 2, caractérisé par le fait qu'à l'étape a), on met en oeuvre la quinone à l'état immobilisé sur un support insoluble par l'intermédiaire d'un polymère à fonctions amine et qu'à l'étape c), on dose l'anticorps monoclonal présent dans sa forme complexée au produit de réaction amine-quinone.

4. Procédé selon la revendication 3, caractérisé par le fait qu'à l'étape c), on fait réagir sur l'anticorps monoclonal présent dans sa forme complexée, un anticorps dirigé contre lui et marqué par un élément dosable, et après réaction, on détermine la quantité dudit anticorps marqué qui s'est fixée audit anticorps monoclonal.

5. Procédé selon l'une des revendications 1 et 2, caractérisé par le fait qu'à l'étape a), on met en oeuvre la quinone à l'état libre et qu'à l'étape c), on dose l'anticorps monoclonal dans sa forme libre.

6. Procédé selon la revendication 5, caractérisé par le fait qu'à l'étape c), on introduit dans le milieu, une quantité connue du composé résultant de la réaction de l'amine considérée et de la quinone considérée, immobilisée sur un support insoluble par l'intermédiaire d'un polymère à fonctions amine, ainsi qu'un anticorps dirigé contre l'anticorps monoclonal et marqué par un élément dosable, et, après réaction, on détermine la quantité de l'anticorps marqué qui qui s'est fixée sur ledit support portant le produit de réaction quinone-amine.

7. Procédé selon la revendication 5,caractérisé par le fait qu'à l'étape c ) l'anticorps monoclonal est de préférence immobilisé sur un support et on ajoute au milieu un conjugué enzyme-amine puis on dose l'activité enzymatique soit sur le support, soit en phase liquide.

8. Procédé selon la revendication 7,caractérisé par le fait que l'on utilise,comme enzyme,une peroxydase.

9. Procédé selon l'une des revendications 1 à 8,caractérisé par le fait qu'il est appliqué au dosage des amines primaires.

10. Procédé selon la revendication 9, caractérisé par le fait qu'il est appliqué au dosage de l'histamine.

11. Procédé selon l'une des revendications 1 à 10,caractérisé par le fait qu'à l'étape a) on utilise,comme quinone, la p-benzoquinone.

12. Anticorps monoclonaux spécifiques du produit résultant de la réaction d'une quinone avec une amine primaire ou secondaire .

13. Anticorps monoclonaux selon la revendication 12 spécifiques du produit résultant de la réaction d'une amine primaire sur la p-benzoquinone.

14. Anticorps monoclonaux selon l'une des revendications 12 et 13,spécifiques du composé résultant de la réaction de la p-benzoquinone et de l'histamine.

15. Anticorps monoclonaux selon la revendication 14, ayant les caractéristiques de ceux secrétés par la lignée d'hybridome dont les échantillons ont été déposés à la CNCM sous le n° I-421.

16. Hybridomes secréteurs d'un anticorps monoclonal selon l'une des revendications 12 à 14.

17. Hybridomes ayant les caractéristiques de ceux appartenant à la lignée cellulaire dont des échantillons ont été déposés à la CNCM sous le n° I-421.

18. Ensemble de réactifs pour l'application du procédé tel que défini à l'une des revendications 1 à 11,caractérisé par le fait qu'il comporte:
- une quinone :
- un anticorps monoclonal spécifique du produit résultant de la réaction de la quinone avec l'amine

à doser ; et
- un système pour le dosage de l'anticorps monoclonal.

**19.** Ensemble selon la revendication 18, pour l'application du procédé tel que défini à la revendication 4, caractérisé par le fait que la quinone est immobilisée sur un support insoluble par l'intermédiaire d'un polymère à fonctions amine et que le système pour le dosage de l'anticorps monoclonal comprend un anticorps dirigé contre lui et marqué par un élément dosable.

**20.** Ensemble selon la revendication 18, pour l'application du procédé tel que défini à la revendication 6, caractérisé par le fait que la quinone est une quinone libre et que le système pour le dosage de l'anticorps monoclonal comprend un composé résultant de la réaction de l'amine à doser et de la quinone considérée immobilisée sur un support insoluble par l'intermédiaire d'un polymère à fonctions amine, ainsi qu'un anticorps dirigé contre ledit anticorps monoclonal et marqué par un élément dosable.

**21.** Ensemble selon la revendication 18, pour l'application du procédé tel que défini à la revendication 7, caractérisé par le fait que la quinone est une quinone libre et que le système pour le dosage de l'anticorps monoclonal comprend un conjugué enzyme-amine.

**22.** Ensemble selon l'une des revendications 18 à 21, caractérisé par le fait qu'il comprend un composé susceptible de bloquer les fonctions réactives de la quinone.

## Claims

**1.** A method for immunological determination of amines in biological media, wherein:
a) the amine present in the sample to be studied is reacted with a quinone;
b) a monoclonal antibody which specifically recognizes the amine-quinone reaction product formed is then introduced into the medium; and
c) determination is carried out of one of the forms of the monoclonal antibody present.

**2.** The method as claimed in claim 1 wherein, between the stage a) and the stage b), blocking of the remaining reactive groups of the quinone is performed.

**3.** The method as claimed in one of claims 1 and 2 wherein, in the stage a), the quinone is used in the immobilized state on insoluble support, the immobilization being achieved by the use of a polymer having an amine groups, and wherein, in the stage c), determination is carried out of the monoclonal antibody present in its complexed form with the amine-quinone reaction product.

**4.** The method as claimed in claim 3 wherein, in the stage c), the monoclonal antibody present in its complexed form is reacted with an antibody directed against itself and labeled with a component which can be measured and, after the reaction, the determination is carried out of the amount of the said labeled antibody which has bound to the said monoclonal antibody.

**5.** The method as claimed in one of claims 1 and 2 wherein, in the stage a), the quinone is used in the free state, and wherein, in the stage c), the monoclonal antibody is determined in its free state.

**6.** The method as claimed in claim 5 wherein, in the stage c), a known amount of compound resulting from the reaction of the amine in question with the quinone in question, the latter being immobilized on an insoluble support via a polymer having amine groups, is introduced into the medium, together with an antibody directed against the monoclonal antibody and labeled with a measurable component, and, after the reaction, determination is carried out of the amount of labeled antibody which has bound to the said support bearing the quinone-amine reaction product.

**7.** The method as claimed in claim 5 wherein, in the stage c), the monoclonal antibody is preferably immobilized on a support, an enzyme-amine conjugate is added to the medium and the enzyme activity is then determined, either on the support or in the liquid phase.

**8.** The method as claimed in claim 7, wherein a peroxidase is used as the enzyme.

9. The method as claimed in one of claims 1 to 8, which is applied to the determination of primary amines.

10. The method as claimed in claim 9, which is applied to the determination of histamine.

11. The method as claimed in one of claims 1 to 10 wherein, in the stage a), p-benzoquinone is used as the quinone.

12. The monoclonal antibodies specific for the product resulting from the reaction of a quinone with a primary or secondary amine.

13. The monoclonal antibodies as claimed in claim 12, which are specific for the product resulting from the reaction of a primary amine with p-benzoquinone.

14. The monoclonal antibodies as claimed in one of claims 12 and 13, which are specific for the compound resulting from the reaction of p-benzoquinone and histamine.

15. The monoclonal antibodies as claimed in claim 14, having the characteristics of those secreted by the hybridoma cell line, the samples of which have been filed with the CNCM under the no. I-421.

16. Hybridomas which secrete a monoclonal antibody as claimed in one of claims 12 to 14.

17. Hybridomas having the characteristics of those belonging to the cell line, the samples of which have been filed with the CNCM under the no. I.421.

18. A kit of reagents for the application of the method as defined in one of claims 1 to 11, which incorporates:
- a quinone;
- a monoclonal antibody specific for the product resulting from the reaction of quinone with the amine to be determined; and
- a system for the determination of the monoclonal antibody.

19. The kit as claimed in claim 18, for the application of the method as defined in claim 4, in which the quinone is immobilized on an insoluble support via a polymer having amino groups and in which the system for determining the monoclonal antibody comprises an antibody directed against itself and labeled with a component which can be measured.

20. The kit as claimed in claim 18 for application of the method as defined in claim 6, in which the quinone is a free quinone and in which the system for determining the monoclonal antibody comprises a compound resulting from the reaction of the amine to be determined and the quinone in question, the latter being immobilized on an insoluble support via a polymer having amine groups, together with an antibody directed against the monoclonal antibody and labeled with a measurable component.

21. The kit as claimed in claim 18 for the application of the process as defined in claim 7, in which the quinone is a free quinone and the system for determining the monoclonal antibody comprises an enzyme-amine conjugate.

22. The kit as claimed in one of claims 18 to 21, which comprises a compound capable of blocking the reactive groups of the quinone.

## Ansprüche

1. Verfahren zur immunologischen Bestimmung von Aminen in biologischem Milieu, dadurch gekennzeichnet, daß
a) das in der zu untersuchenden Probe vorhandene Amin mit einem Chinon zur Reaktion gebracht wird;
b) danach in das Milieu ein monoklonaler Antikörper eingeführt wird, der spezifisch das bei der

Amin/Chinon-Reaktion gebildete Produkt erkennt;
c) eine der vorhandenen Formen des monoklonalen Antikörpers bestimmt wird.

2. Verfahren nach Anspruch 1, dadurch gekennzeichnet, daß zwischen Schritt a) und Schritt b) am Chinon verbliebene reaktive Funktionen blockiert werden.

3. Verfahren nach einem der Ansprüche 1 und 2, dadurch gekennzeichnet, daß bei Schritt a) das Chinon in auf einen unlöslichen Träger durch die Vermittlung eines Polymers mit Aminfunktionen fixiertem Zustand eingesetzt wird, und daß bei Schritt c) der monoklonale Antikörper, der in mit dem Amin/Chinon-Reaktionsprodukt komplexierter Form vorliegt, bestimmt wird.

4. Verfahren nach Anspruch 3, dadurch gekennzeichnet, daß bei Schritt c) auf dem in komplexierter Form vorliegenden monoklonalen Antikörper ein Antikörper zur Reaktion gebracht wird, der gegen diesen gerichtet und mit einem bestimmbaren Faktor markiert ist, und nach der Reaktion die Menge des markierten Antikörpers bestimmt wird, der auf dem monoklonalen Antikörper fixiert ist.

5. Verfahren nach einem dar Ansprüche 1 und 2, dadurch gekennzeichnet, daß bei Schritt a) das Chinon in freiem Zustand eingesetzt wird und daß bei Schritt c) der monoklonale Antikörper in freier Form bestimmt wird.

6. Verfahren nach Anspruch 5, dadurch gekennzeichnet, daß bei Schritt c) in das Milieu eine bekannte Menge der bei der Reaktion des betreffenden Amins und des betreffenden Chinons gebildeten Verbindung, fixiert auf einen unlöslichen Träger durch die Vermittlung eines Polymers mit Aminfunktionen, eingebracht wird, sowie ein Antikörper, der gegen den monoklonalen Antikörper gerichtet und mit einem bestimmbaren Faktor markiert ist, und nach der Reaktion die Menge des markierten Antikörpers bestimmt wird, der auf dem Träger fixiert ist, der das Chinon/Amin-Reaktionsprodukt trägt,

7. Verfahren nach Anspruch 5, dadurch gekennzeichnet, daß bei Schritt c) der monoklonale Antikörper vorzugsweise auf einen Träger fixiert ist und dem Milieu ein Enzym/Amin-Konjugat zugesetzt, dann die Enzymaktivität entweder auf dem Träger oder in flüssiger Phase bestimmt wird.

8. Verfahren nach Anspruch 7, dadurch gekennzeichnet, daß als Enzym eine Peroxidase verwendet wird.

9. Verfahren nach einem der Ansprüche 1 bis 8, dadurch gekennzeichnet, daß es zur Bestimmung von primären Aminen eingesetzt wird.

10. Verfahren nach Anspruch 9, dadurch gekennzeichnet, daß es zur Bestimmung von Histamin eingesetzt wird.

11. Verfahren nach einem der Ansprüche 1 bis 10, dadurch gekennzeichnet, daß bei Schritt a) als Chinon p-Benzochinon verwendet wird.

12. Monoklonale Antikörper, spezifisch für das Produkt, das bei der Reaktion eines Chinons mit einem primären oder sekundären Amin gebildet wird.

13. Monoklonale Antikörper nach Anspruch 12, spezifisch für das Produkt, das bei der Reaktion eines primären Amins mit p-Benzochinon gebildet wird.

14. Monoklonale Antikörper nach einem der Ansprüche 12 und 13, spezifisch für die Verbindung, die bei der Reaktion von p-Benzochinon mit Histamin gebildet wird.

15. Monoklonale Antikörper nach Anspruch 14, dadurch gekennzeichnet, daß sie aus der Hybridom-Linie stammen, deren Proben beim CNCM unter Nr. I-421 hinterlegt wurden.

16. Hybridome, die einen monoklonaler Antikörper nach einem der Ansprüche 12 bis 14 absondern.

17. Hybridome, dadurch gekennzeichnet, daß sie der Zellinie angehören, deren Proben beim CNCM unter Nr. I-421 hinterlegt wurden.

18. Reagenzien-Kombination zur Anwendung in einem Verfahren, das nach einem der Ansprüche 1 bis 11 definiert ist, dadurch gekennzeichnet, daß sie
    - ein Chinon;
    - einen monoklonalen Antikörper, der für das bei der Reaktion des Chinons mit dem zu bestimmenden Amin spezifisch ist; und
    - ein System zur Bestimmung des monoklonalen Antikörpers umfaßt.

19. Kombination nach Anspruch 18 zur Anwendung in einem Verfahren, das nach Anspruch 4 definiert ist, dadurch gekennzeichnet, daß das Chinon auf einen unlöslichen Träger durch die Vermittlung eines Polymers mit Aminfunktionen fixiert wird, und daß das System zur Bestimmung des monoklonalen Antikörpers einen Antikörper umfaßt, der gegen diesen gerichtet und mit einem bestimmbaren Faktor markiert ist.

20. Kombination nach Anspruch 18 zur Anwendung in einem Verfahren, das nach Anspruch 6 definiert ist, dadurch gekennzeichnet, daß das Chinon ein freies Chinon ist, und daß das System zur Bestimmung des monoklonalen Antikörpers eine bei der Reaktion des zu bestimmenden Amins mit dem betreffenden Chinon gebildeten Verbindung, fixiert auf einen unlöslichen Träger durch die Vermittlung eines Polymers mit Aminfunktionen, umfaßt sowie einen Antikörper, der gegen den monoklonalen Antikörper gerichtet und mit einem bestimmbaren Faktor markiert ist.

21. Kombination nach Anspruch 18 zur Anwendung in einem Verfahren, das nach Anspruch 7 definiert ist, dadurch gekennzeichnet, daß das Chinon ein freies Chinon ist, und daß das System zur Bestimmung des monoklonalen Antikörpers ein Enzym/Amin-Konjugat umfaßt.

22. Kombination nach einem der Ansprüche 18 bis 21, dadurch gekennzeichnet, daß sie eine Verbindung umfaßt, die befähigt ist, die reaktiven Funktionen des Chinons zu blockieren.

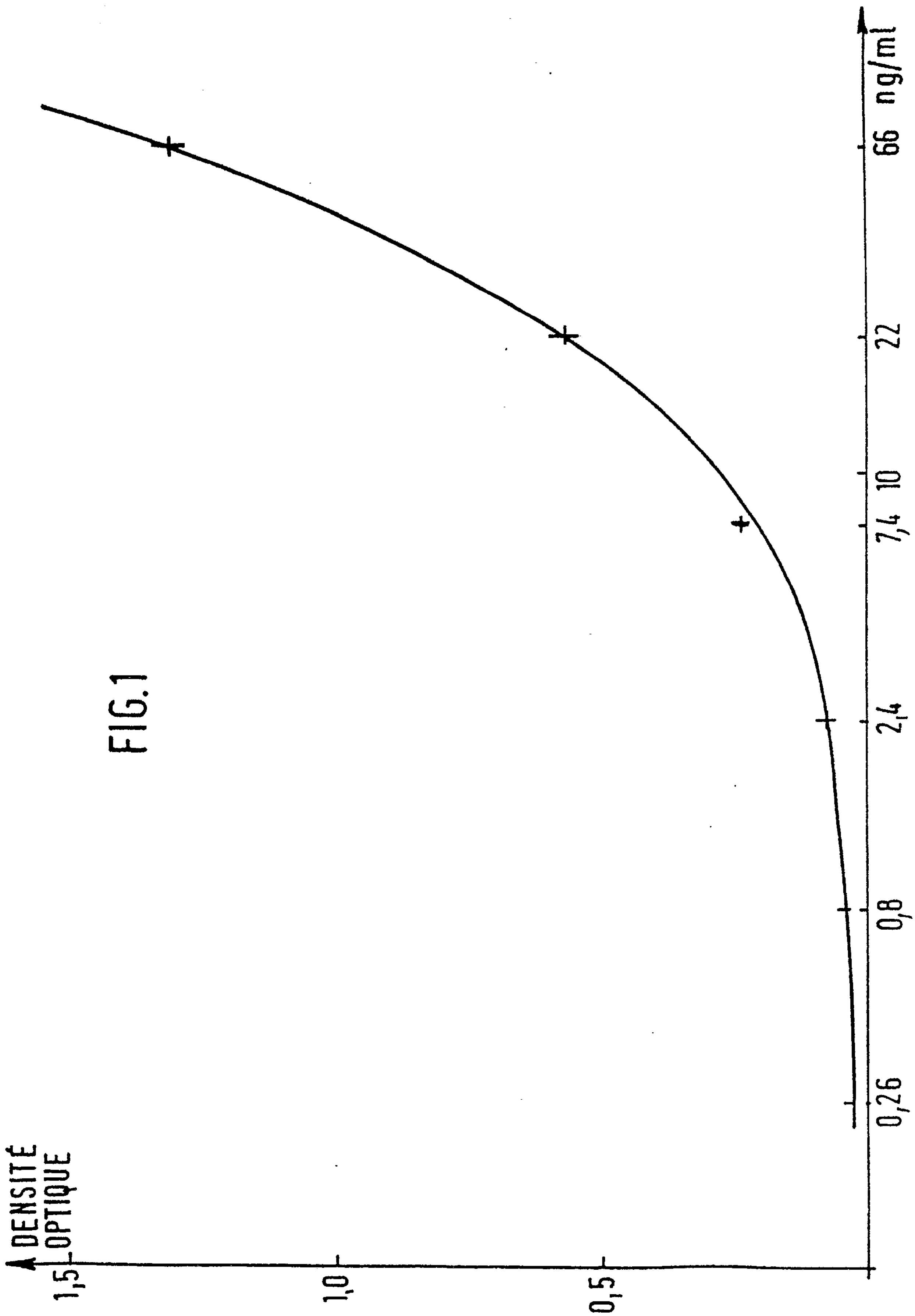


FIG.1

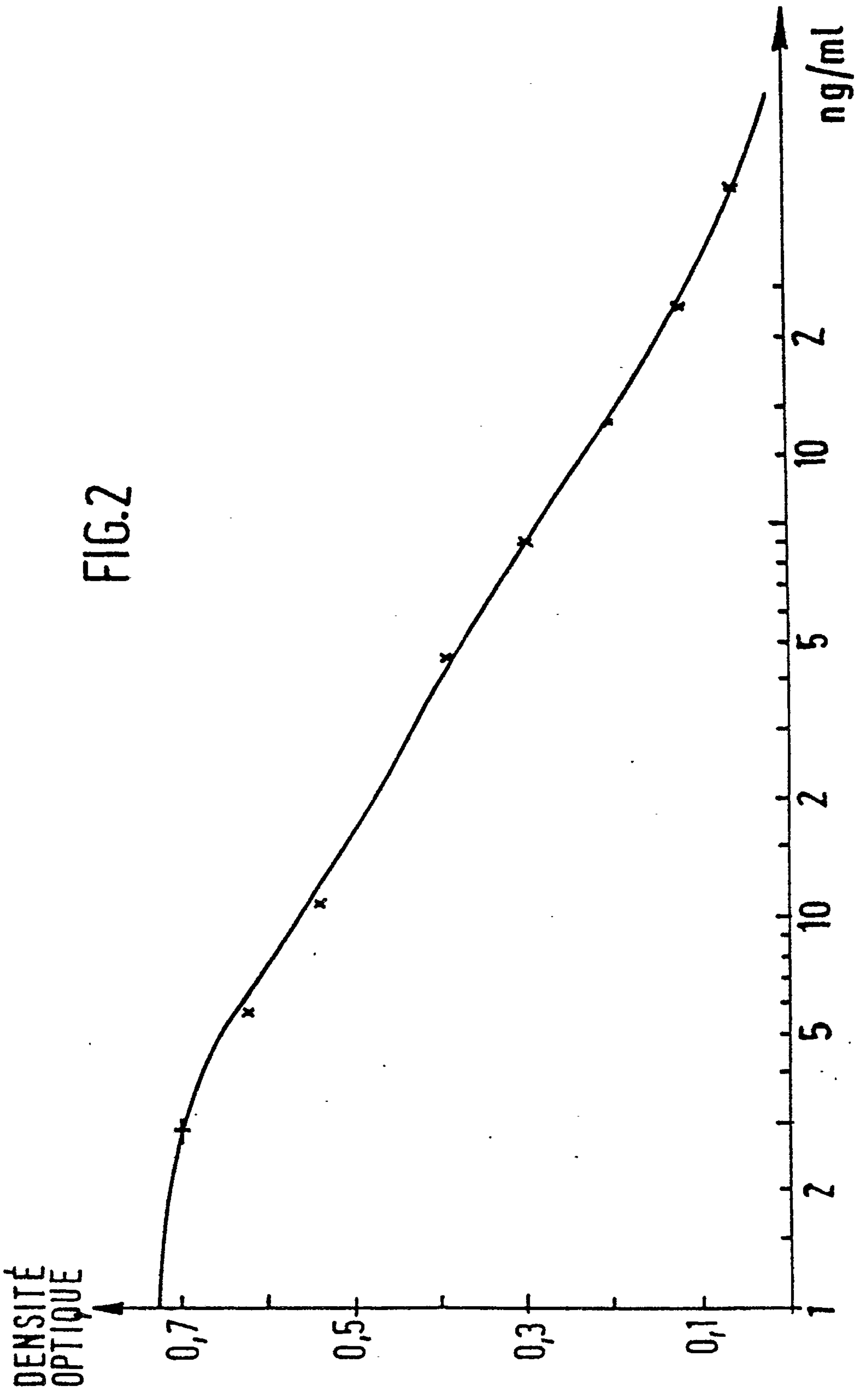


FIG.2

FIG.3

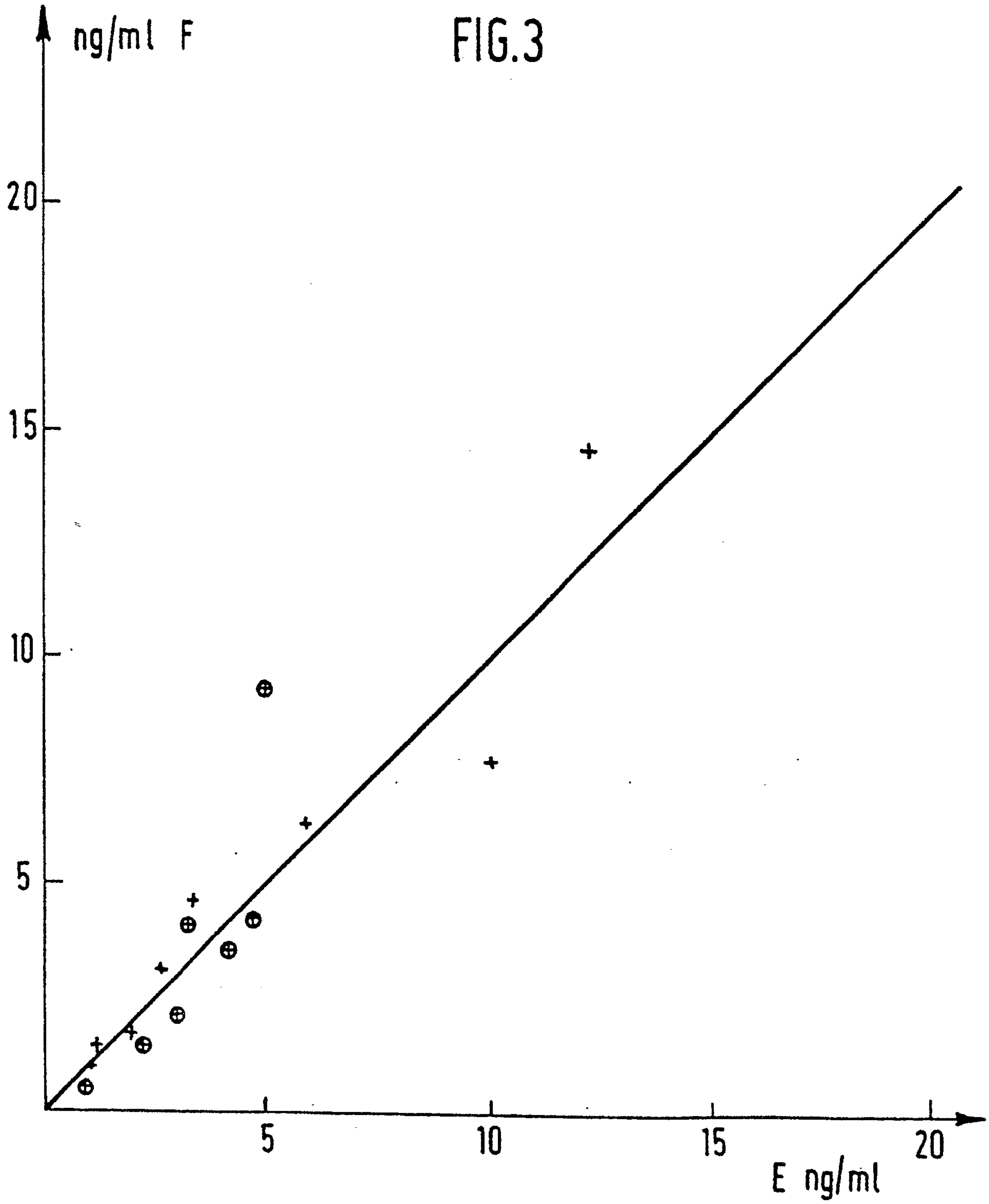

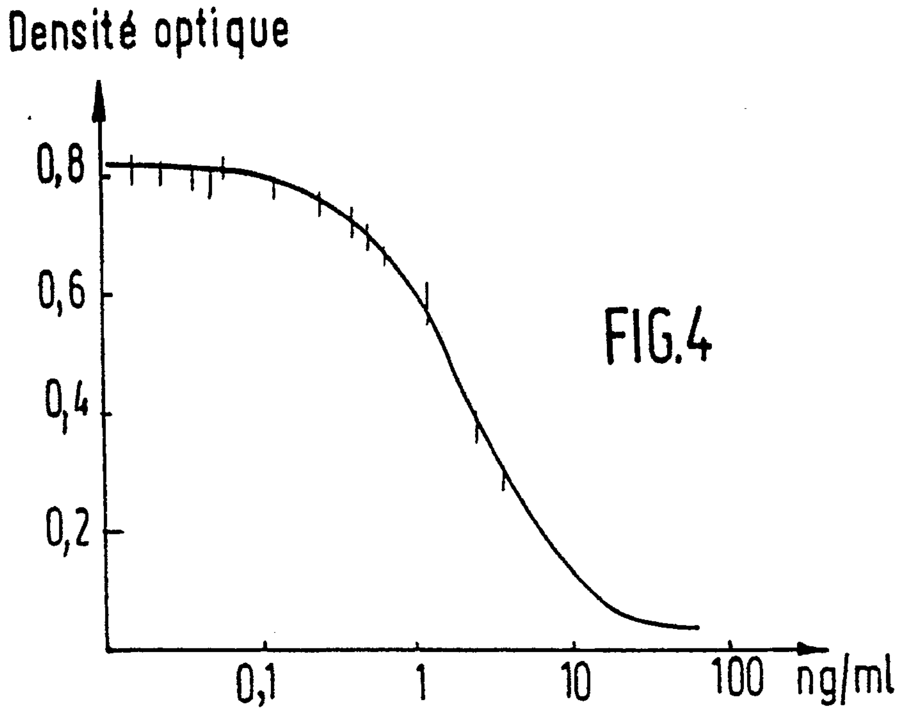
Densité optique
0,8
0,6
0,4
0,2
0,1
1
10
100
ng/ml

FIG.4

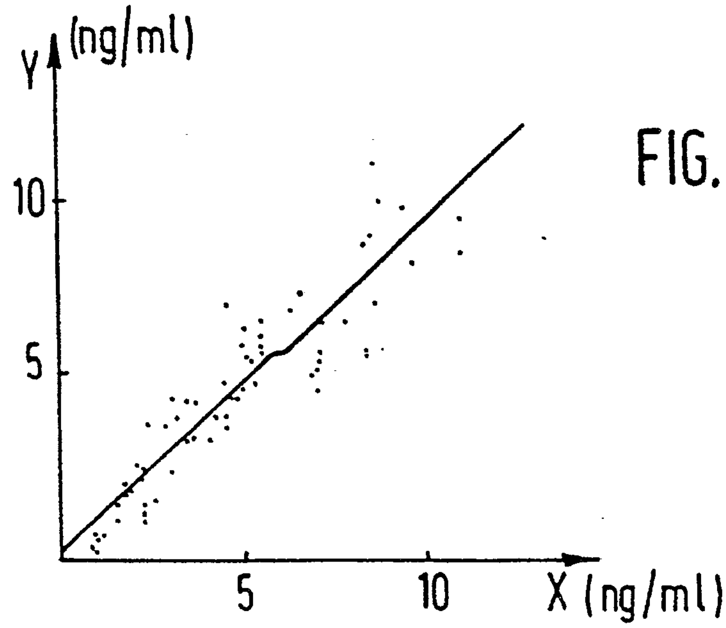
Y (ng/ml)
10
5
5
10
X (ng/ml)

FIG. 5